# EUROPÄISCHE PATENTSCHRIFT

(11) **EP 0 674 943 B1**
(45) Veröffentlichungstag und Bekanntmachung des Hinweises auf die Patenterteilung: **16.09.1998**
(21) Anmeldenummer: 95104058.3
(22) Anmeldetag: 20.03.1995
(51) Int. Cl.: B01J 23/20, C07C 209/18

(54) **Nioboxid- Hydrat- und Tantaloxid-Hydrat-Katalysatoren, ihre Herstellung und ein Verfahren zur Herstellung von N-alkylierten Anilinen mit diesen Katalysatoren**
Hydrated tantalumoxide or niobiumoxide catalysts, their preparation and process for preparing N-alkylated anilines therewith
Catalyseurs en oxyde hydraté de tantale ou de niobium, leur préparation et procédé de préparation d'anilines N-alkylés les utilisant

(30) Priorität: 31.03.1994 DE 4411234
(43) Veröffentlichungstag der Anmeldung: 04.10.1995
(73) Patentinhaber: BAYER AG, 51368 Leverkusen (DE)
(72) Erfinder: Glock, Volker, Dr., D-47800 Krefeld (DE); Pentling, Ursula, Dr., D-47906 Kempen (DE); Pennemann, Bernd, Dr., D-51061 Köln (DE); Jentsch, Joerg-Dietrich, Dr., D-45468 Mülheim (DE); Zirngiebl, Eberhard, Dr., D-51061 Köln (DE); Köller, Horst, Dr., D-47802 Krefeld (DE)

(56) Entgegenhaltungen:
- EP-A- 0 135 145
- EP-A- 0 433 811
- EP-A- 0 509 493

## Beschreibung

Die Erfindung betrifft Nioboxid-Hydrat- und Tantaloxid-Hydrat-Katalysatoren mit besonderen Poren und Seitenbruchhärten, ihre Herstellung und ein Verfahren zur Herstellung von N-alkylierten Anilinen mit diesen Katalysatoren.

N-alkylierte aromatische Amine sind Ausgangsprodukte zur Herstellung von Farbstoffen, Schädlingsbekämpfungsmitteln, Urethanen, Arzneimitteln und Pflanzenschutzmitteln. Ferner dienen sie als Mineralölzusätze und als Zusätze für Lacke und andere polymere Systeme.

Es sind verschiedene Verfahren zur Herstellung von sekundären und tertiären aromatischen Aminen bekannt. So ist bereits bekannt, zur Herstellung von N-alkylierten aromatischen Aminen aromatische Amine mit Alkanolen oder Dialkylethern an heterogenen Katalysatoren in der Gasphase umzusetzen.

Der Stand der Technik zur Herstellung von N-alkylierten Anilinen an heterogenen Katalysatoren ist beispielsweise in der EP-A 433 811 beschrieben. Dort ist auch der Einsatz von Nioboxid-Hydrat und Tantaloxid-Hydrat als Katalysator beschrieben.

Zur Herstellung von Granulaten, Extrudaten oder Kugeln kann der Katalysator auf einen Träger aufgezogen oder versehen mit einem Bindemittel granuliert oder extrudiert werden. Er kann auch mit einem Gleitmittel versehen und durch Tablettierung in eine stückige Form gebracht werden.

Bezüglich des Einsatzes des Katalysators in einem Festbett-Gasphasenreaktor ist es wesentlich, daß der granulierte Katalysator ausreichende Festigkeit aufweist. Es ist bekannt, daß Katalysatoren mit nicht ausreichender Festigkeit aufgrund des Eigengewichtes, des Druckverlustes im Reaktor, der thermischen Belastung und aus anderen Ursachen zerfallen können, was dann zu stark ungleichen Druckverlusten in Festbetten und schließlich zur Verstopfung des Reaktors führen kann. Wenig feste Katalysatoren haben deshalb nur eine geringe Lebensdauer und sind für eine technische Anwendung nicht geeignet. Ein Maß für die Festigkeit ist die sog. Seitenbruchhärte. Die Bestimmung der Seitenbruchhärte kann mit üblichen Meßgeräten erfolgen, z.B. mit den von der Fa. Schleuniger in den Handel gebrachten.

Katalysatoren mit Bruchhärten unter 20 N sind für den technischen Einsatz nicht geeignet, da sie erfahrungsgemäß den bei der Befüllung und dem Betrieb eines technischen Reaktors auftretenden Belastungen nicht standhalten (siehe Vergleichsbeispiel 2). Es ist bekannt, daß die Aktivität eines mechanisch verformten Katalysators und seine Festigkeit in einem reziproken Verhältnis stehen und üblicherweise eine Erhöhung der Festigkeit eine Abnahme der katalytischen Aktivität bewirkt.

Es wurden nun Nioboxid-Hydrat- und Tantaloxid-Hydrat-Katalysatoren in stückiger Form mit einem Gehalt an inertem Feststoff gefunden, die Poren mit Durchmessern in dem Bereich von 0,4 bis 10 000 nm enthalten und dadurch gekennzeichnet sind, daß das Volumen der Poren des Katalysators mit einem Durchmesser von 100 bis 1 000 nm mindestens 30 % des gesamten Porenvolumens des Katalysators ausmacht. Bevorzugt beträgt der Anteil dieser Poren mindestens 33 % des gesamten Porenvolumens und besonders bevorzugt mindestens 36 %.

Erfindungsgemäße Katalysatoren weisen im allgemeinen Seitenbruchhärten von über 20 N, bevorzugt über 25 N auf.

Die Bestimmung des Porenvolumens und der Porendurchmesserverteilung kann auf bekannte Weise, beispielsweise mit einem Quecksilberporosimeter erfolgen.

Erfindungsgemäße Katalysatoren können hergestellt werden, indem man Nioboxid-Hydrat oder Tantaloxid-Hydrat mit einem inerten Feststoff, beispielsweise Graphit, vermischt und solche Gemische in stückige feste Form bringt, z.B. durch Tablettieren mit einer Tablettierpresse. Um erfindungsgemäße Katalysatoren zu erhalten, ist es im allgemeinen erforderlich, den Druck in der Vorrichtung zur Herstellung von Katalysatoren in stückiger Form so einzustellen, daß das Volumen der Poren des Katalysators mit einem Durchmesser von 100 bis 1 000 nm mindestens 30 % des gesamten Porenvolumens beträgt. Zur Einstellung eines geeigneten Druckes in der Vorrichtung zur Herstellung von Katalysatoren in stückiger Form können vorher routinemäßige Reihenversuche durchgeführt werden, wobei man Drucke beispielsweise im Bereich von 500 bis 50 000 kg/cm² variieren kann und berücksichtigen sollte, daß die Anwendung höherer Drucke den Anteil von Poren mit Durchmessern im Bereich 100 bis 1 000 nm erniedrigt.

Die so erhaltenen Nioboxid-Hydrat- und Tantaloxid-Hydrat-Katalysatoren weisen die für einen technischen Einsatz erforderliche Seitenbruchhärte und gleichzeitig eine deutlich höhere Aktivität auf als bisher bekannte Katalysatoren. Die erfindungsgemäßen Katalysatoren können beispielsweise für Dehydrierungen, oxidative Dehydrierungen, Ammonoxidationen, Polymerisationen, Isomerisierungen, Zersetzungen (beispielsweise von NOₓ), Fischer-Tropsch-Synthesen, Hydrierungen und Alkylierungen, insbesondere N-Alkylierungen, eingesetzt werden.

Es wurde weiterhin gefunden, daß erfindungsgemäße Katalysatoren besonders geeignet sind für die Herstellung von N-Alkylanilinen aus Anilinen und Alkanolen. Beispielsweise kann man in dieses Verfahren als Aniline solche der Formel (I) 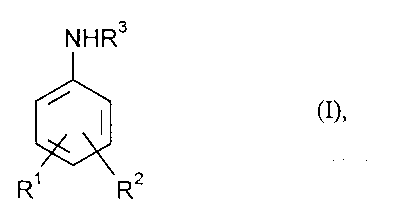 in der
- R¹ und R²: unabhängig voneinander Wasserstoff, C₁-C₄-Alkyl, C₁-C₄-Alkoxy, Fluor, Chlor, Brom oder Cyano und
- R³: Wasserstoff, C₁-C₁₂-Alkyl oder C₃-C₇-Cycloalkyl bedeuten
und als Alkanole solche der Formel

R⁴-OH (II),

in der
- R⁴: C₁-C₁₂-Alkyl oder C₃-C₇-Cycloalkyl bedeuten
einsetzen und so N-Alkylaniline der Formel (III) erhalten 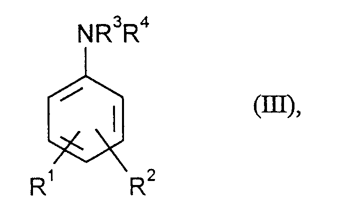 in der
- R¹ bis R⁴: die oben angegebene Bedeutung haben.

Man kann diese Umsetzung in der Sumpfphase oder Gasphase beispielsweise bei Temperaturen von 160 bis 400°C und mit Molverhältnissen von Alkanol zu Anilinen von 0,5 bis 10:1 durchführen. Das vorbeschriebene Verfahren zur Herstellung von N-Alkylanilinen hat den überraschenden Vorteil, daß es die Realisierung hoher Raum-Zeit-Ausbeuten zuläßt und die Anteile von nicht umgesetzten Edukten gering gehalten werden können. Der eingesetzte Katalysator weist eine für ein technisch auszuübendes Verfahren erforderliche Festigkeit auf.

Die Prozentangaben beziehen sich, soweit nichts anderes angegeben ist, auf das Gewicht.

### Beispiele

### Vergleichsbeispiel 1

In ein Edelstahl-Reaktionsrohr von 25 mm Innendurchmesser wurden 118,5 g tablettierten Nioboxidhydrats (Durchmesser 6,4 mm) eingefüllt, bei dem der Anteil der Poren mit einem Durchmesser von 100 bis 1 000 nm 24 % des gesamten Porenvolumens und die mittlere Seitenbruchhärte bei 148 N lag. Durch diese Katalysatorschicht wurde bei 261°C ein Gasgemisch aus Ethanol und unsubstituiertem Anilin im Molverhältnis 1:4 mit einer Geschwindigkeit von 1,35 g/ml·h geleitet. Das im Verlauf von 48 Stunden entstandene Reaktionsprodukt hatte laut Gaschromatographie die Zusammensetzung:

| | |
|---|---|
| Anilin | 17,47 Flächen-% |
| N-Ethylanilin | 32,51 Flächen-% |
| N,N-Diethylanilin | 47,90 Flächen-% |
| Nebenprodukte | 2,12 Flächen-% |

### Vergleichsbeispiel 2

Nioboxid-Hydrat wurde unter Zusatz von 3,5 % Graphit auf einer Laborpresse zu Tabletten verpreßt, die einen Durchmesser von 5 mm, eine mittlere Seitenbruchhärte von 14 N und einen Anteil von Poren mit einem Durchmesser von 100 bis 1 000 nm von 56 % des gesamten Porenvolumens aufwiesen. 89 g des Katalysators wurden unter den Bedingungen des Vergleichsbeispiels 1, jedoch bei einer Temperatur von 265°C, getestet und nach 168 Stunden ein Reaktionsgemisch mit folgender Zusammensetzung erhalten:

| | |
|---|---|
| Anilin | 8,22 Flächen-% |
| N-Ethylanilin | 39,09 Flächen-% |
| N,N-Diethylanilin | 51,12 Flächen-% |
| Nebenprodukte | 1,57 Flächen-% |

Die Festigkeit dieses Katalysators war für einen technischen Einsatz nicht ausreichend, denn bereits bei einer Höhe des Festbettes von 3 m zerbrach er unter seinem Eigengewicht.

### Beispiel 1

Nioboxid-Hydrat wurde unter Zusatz von 4 % Graphit so zu Tabletten auf einer technischen Presse verpreßt, daß die Tabletten einen Durchmesser von 5 mm, eine mittlere Seitenbruchhärte von 41 N und einen Anteil von Poren mit einem Durchmesser von 100 bis 1 000 nm von 42 % des gesamten Porenvolumens aufwiesen. 106,3 g des Katalysators wurden unter den Bedingungen des Vergleichsbeispiels 1 getestet. Dabei wurde ein Reaktionsgemisch mit folgender Zusammensetzung erhalten:

| | |
|---|---|
| Anilin | 7,23 Flächen-% |
| N-Ethylanilin | 35,05 Flächen-% |
| N,N-Diethylanilin | 55,66 Flächen-% |
| Nebenprodukte | 2,06 Flächen-% |

Im Gegensatz zu Vergleichsbeispiel 2 wies dieser Katalysator für den technischen Einsatz eine ausreichende Festigkeit auf, da er bei einer Höhe des Festbettes von 3 m unter seinem Eigengewicht nicht zerbrach.

### Beispiel 2

597 kg des Katalysators aus Beispiel 1 wurden in einem Rohrbündelreaktor aus 350 Rohren mit einem Durchmesser von jeweils 2,5 cm und einer Länge von 3,5 m gefüllt. Über die Katalysatorschicht wurde bei 262°C ein Gemisch aus Ethanol und Anilin im Molverhältnis 1:3 mit einer Geschwindigkeit von 600 kg/h geleitet. Das im Verlauf von 24 Stunden entstandene Reaktionsprodukt hatte laut Gaschromatographie die Zusammensetzung:

| | |
|---|---|
| Anilin | 4,23 Flächen-% |
| N-Ethylanilin | 38,81 Flächen-% |
| N,N-Diethylanilin | 53,99 Flächen-% |
| Nebenprodukte | 2,97 Flächen-% |

Nach 500 Stunden zeigte der Katalysator noch keine nennenswerten mechanischen Defekte.

## Patentansprüche

1. Nioboxid-Hydrat- und Tantaloxid-Hydrat-Katalysatoren in stückiger Form mit einem Gehalt an inertem Feststoff, die Poren mit Durchmessern in dem Bereich von 0,4 bis 10 000 nm enthalten, dadurch gekennzeichnet, daß das Volumen der Poren des Katalysators mit einem Durchmesser von 100 bis 1 000 nm mindestens 30 % des gesamten Porenvolumens des Katalysators ausmacht, und daß die Katalysatoren eine Seitenbruchhärte von über 20 N aufweisen.

2. Katalysatoren nach Anspruch 1, dadurch gekennzeichnet, daß der Anteil der Poren mit einem Durchmesser von 100 bis 1 000 nm mindestens 33 % des gesamten Porenvolumens ausmacht.

3. Katalysatoren nach Ansprüchen 1 und 2, dadurch gekennzeichnet, daß sie eine Seitenbruchhärte von über 25 N aufweisen.

4. Verfahren zur Herstellung von Nioboxid-Hydrat- und Tantaloxid-Hydrat-Katalysatoren gemäß Anspruch 1, wobei man Nioboxid-Hydrat oder Tantaloxid-Hydrat mit einem inerten Feststoff vermischt und solche Gemische unter einem Druck von 500 bis 50 000 kg/cm² in eine stückige Form bringt.

5. Verfahren zur Herstellung von N-Alkylanilinen aus Anilinen und Alkanolen, dadurch gekennzeichnet, daß man dafür Katalysatoren des Anspruchs 1 oder Katalysatoren hergestellt nach Anspruch 4 einsetzt.

## Claims

1. Hydrated niobium oxide and hydrated tantalum oxide catalysts in pellet form containing an inert solid and containing pores having diameters in the range from 0.4 to 10,000 nm, characterized in that the volume of the pores of the catalyst having a diameter of from 100 to 1000 nm makes up at least 30 % of the total pore volume of the catalyst and in that the catalysts have a lateral fracture hardness of over 20 N.

2. Catalysts according to Claim 1, characterized in that the proportion of the pores having a diameter of from 100 to 1000 nm makes up at least 33 % of the total pore volume.

3. Catalysts according to Claim 1 or 2, characterized in that they have a lateral fracture hardness of over 25 N.

4. Process for preparing hydrated niobium oxide and hydrated tantalum oxide catalysts according to Claim 1, wherein hydrated niobium oxide or hydrated tantalum oxide is mixed with an inert solid and such mixtures are converted into a pellet form at a pressure of from 500 to 50 000 kg/cm².

5. Process for preparing N-alkylanilines from anilines and alkanols, characterized in that catalysts of Claim 1 or catalysts prepared according to Claim 4 are used therefor.

## Revendications

1. Catalyseur à base d'oxyde hydraté de niobium ou d'oxyde hydraté de tantale, à l'état de fragments contenant une matière solide inerte, avec des pores dont le diamètre se situe dans l'intervalle de 0,4 à 10 000 nm, qui se caractérise en ce que le volume des pores du catalyseur dont le diamètre va de 100 à 1 000 nm représente au moins 30 % du volume de pores total du catalyseur, et en ce que le catalyseur a une dureté de cassure latérale supérieure à 20 N.

2. Catalyseurs selon revendication 1 caractérisés en ce que la proportion des pores de diamètre 100 à 1 000 nm représente au moins 33 % du volume de pores total.

3. Catalyseurs selon les revendications 1 et 2, caractérisés en ce qu'ils ont une dureté de cassure latérale supérieure à 25 N.

4. Procédé de préparation des catalyseurs à base d'oxyde de niobium hydraté ou d'oxyde de tantale hydraté selon revendication 1, selon lequel on mélange un oxyde de niobium hydraté ou un oxyde de tantale hydraté avec une matière solide inerte et on met le mélange sous la forme de fragments sous une pression de 500 à 50 000 kg/cm².

5. Procédé pour la préparation de N-alkylanilines à partir d'anilines et d'alcanols, caractérisé en ce que l'on utilise à cet effet des catalyseurs selon revendication 1 ou des catalyseurs préparés selon revendication 4.
